(19) Europäisches Patentamt
European Patent Office
Office européen des brevets

(11) Veröffentlichungsnummer: **0 046 294**
**A1**

(12) # EUROPÄISCHE PATENTANMELDUNG

(21) Anmeldenummer: **81106387.4**

(51) Int. Cl.³: **C 07 D 207/26**

(22) Anmeldetag: **17.08.81**

(30) Priorität: **18.08.80 DE 3031118**

(43) Veröffentlichungstag der Anmeldung: **24.02.82**
**Patentblatt 82/8**

(84) Benannte Vertragsstaaten: **AT BE CH FR GB IT LI LU NL SE**

(71) Anmelder: **Meditest Institut für medizinisch-pharmazeutische Untersuchungen GmbH & Co. KG, D-7958 Laupheim (DE)**

(72) Erfinder: **Lange, Fritz Walter, Königswieser Strasse 26, D-8035 Gauting (DE)**
Erfinder: **Müller, Jens, Königswieser Strasse 26, D-8035 Gauting (DE)**

(74) Vertreter: **Berg, Wilhelm, Dr. et al, Dr. Berg, Dipl.-Ing. Stapf, Dipl.-Ing. Schwabe, Dr. Dr. Sandmair Mauerkircherstrasse 45, D-8000 München 80 (DE)**

(54) **N,N'-Bis(5-oxo-1-methyl-pyrrolidin-2-yl-essigsäure)-hydrazid, Verfahren zu dessen Herstellung und seine Verwendung als Arzneimittel.**

(57) Die Erfindung betrifft die neue Verbindung N,N'-Bis-(5-oxo-1-methyl-pyrrolidin-2-yl-essigsäure)-hydrazid der Formel (I)

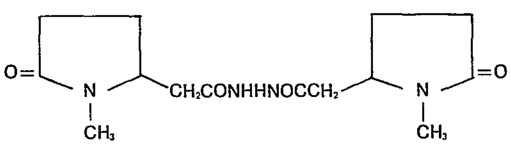

(I)

Verfahren zu deren Herstellung und ihre Verwendung als Arzneimittel, insbesondere als Psychopharmakon (nootrope Substanz).

EP 0 046 294 A1

Die Erfindung betrifft die neue Verbindung N,N'-Bis (5-oxo-1-methyl-pyrrolidin-2-yl-essigsäure)-hydrazid der Formel (I)

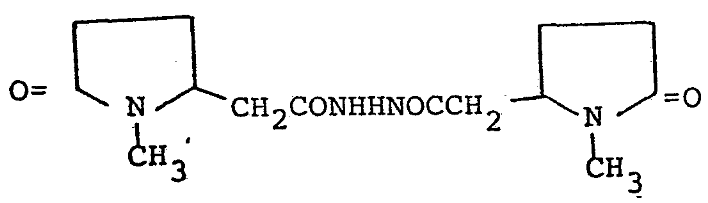

(I)

Verfahren zu deren Herstellung und ihre Verwendung als Arzneimittel, insbesondere als Psychopharmakon (nootrope Substanz).

Es wurde überraschenderweise gefunden, daß das erfindungsgemäße N,N'-Bis(5-oxo-1-methyl-pyrrolidin-2-yl-essigsäure) -hydrazid eine bessere Wirksamkeit als Psychopharmakon aufweist, als vergleichbare bekannte Wirkstoffe, wie z.B. 2-oxo-1-pyrrolidinacetamid (Piracetam). Therapeutische Wirkungen wie Piracetam weisen auch die Verbindungen der DAS 24 40 633, DOS 27 57 680, sowie der DOS 27 45 907 auf. Besonders überraschend ist, daß sich die erfindungsgemäße Verbindung der Formel (I) noch bei bruchteiliger Dosierung dem Piracetam vor allem hinsichtlich seiner nootropen Wirkung überlegen erweist.

Die Überlegenheit der Verbindung (I) zeigt sich besonders deutlich im sogenannten Passiv-Avoidance-Test, wobei das Lernvermögen der mit der erfindungsgemäßen Verbindung behandelten Versuchstiere signifikant besser war, als der mit Piracetam behandelten Tiere. Überraschend ist auch die gute Schutzwirkung der erfindungsgemäßen Verbindung gegenüber der durch Elektroschock induzierten Amnesie bei der Maus. Die erfindungsgemäße Verbindung wirkt gegen retrograde und anterograde Amnesie, Ischämien verschiedener Genese und hat nootrope Wirkung; sie dient zur Verhütung, Besserung und/oder Heilung von Krankheiten, die im zerebralen Funktionsgebiet auftreten können, wie Apolexi cerebri, chronische Hirnfunktionsstörungen wie Zerebralsklerose, Gedächtnisminderung nach Durchblutungsstörungen als Folgen des Alkoholismus oder traumatischer Einwirkungen.

Es wurde weiterhin festgestellt, daß die erfindungsgemäße Verbindung eine überlegene pharmakologische Wirksamkeit bei gleichzeitig äußerst geringer Toxizität aufweist.

Für die Umsetzung der Ester der Formel (II) mit Hydrazin benötigt man etwa molare Verhältnisse, d.h. etwa 1 Mol Ester auf 1 Mol Hydrazin. Zweckmäßig arbeitet man jedoch mit einem Hydrazinüberschuß beispielsweise 1,1 bis 1,5 Mol Hydrazin auf 1 Mol Ester. Die Umsetzung erfolgt zweckmäßig unter Rühren in einem Lösungsmittel, beispielsweise Toluol bei etwas erhöhter Temperatur, vorzugsweise zwischen 50 - 60° C.

Vorzugsweise wird die zuerst gebildete Mono-Verbindung nicht isoliert, sondern nach dem Abdestillieren des Lösungsmittels zur Herstellung der erfindungsgemäßen Bis-Verbindung die erforderliche Menge des Esters der Formel II, 1 bis 1,5 Mol, vorzugsweise 1 Mol, zugegeben und die Mischung 10 bis 30 Std. vorzugsweise etwa 20 Std. auf über 50° erhitzt. Die besten Ausbeuten werden bei Temperaturen zwischen 100° und 180° C, vorzugsweise bei etwa 150° C erhalten. Das Ende der Umsetzung ist dadurch erkennbar, daß kein Alkohol mehr abdestilliert.

0046294

Nach Zugabe eines geeigneten Lösungsmittels, vorzugsweise Isopropanol kristallisiert die erfindungsgemäße Verbindung aus, die dann aus dem gleichen Lösungsmittel durch mehrmaliges Umkristallisieren gereinigt werden kann.

Die Herstellung der erfindungsgemäßen Verbindung wird durch folgendes Beispiel näher erläutert:

Beispiel

Eine Lösung von 171,2 g (1 Mol) 1-Methyl-5-oxo-pyrrolidin-2-essigsäuremethylester in 250 ml Toluol wird mit 75 g (1,5 Mol) Hydrazinhydrat (100-prozentig) versetzt. Anschließend wird die Mischung unter Rühren 10 Std. auf 50 - 60° C erhitzt. Nachdem im Vakuum alles Lösungsmittel abdestilliert wurde, werden weitere 171,2 g(1 Mol) 1-Methyl-5-oxo-pyrrolidin-2-essigsäuremethylester hinzugefügt und die Mischung anschliessend 20 Std. auf 150° C erwärmt, wobei das Methanol abdestilliert wird. Das Ende der Reaktion wird dadurch angezeigt, daß kein Methanol mehr abgespalten wird. Das Reaktionsprodukt kristallisiert nach Zugabe von 150 ml Isopropanol aus. Es wird mehrmals aus dem gleichen Lösungsmittel umkristallisiert, wobei weiße Kristalle vom Schmp. 229 - 230° C erhalten werden.

Ausbeute: 139,5 - 155,2 g (45 - 50 % der Theorie).

Die vorliegende Erfindung betrifft auch pharmazeutische Zubereitungen, die neben nichttoxischen, inerten pharmazeutisch geeigneten Trägerstoffen die erfindungsgemäßen Wirkstoffe enthalten.

Die vorliegende Erfindung betrifft ferner pharmazeutische Zubereitungenen in Form von Dosierungseinheiten d.h., daß die Zubereitungen beispielsweise in Form von Tabletten, Dragees, Kapseln, Pillen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Als bevorzugte pharmazeutische Zubereitungen seien Tabletten, Dragees, Kapseln, Pillen, Granulate, Lösungen, Suspensionen und Emulsionen sowie Puder genannt.

Tabletten, Dragees, Kapseln, Pillen und Granulate können den Wirkstoff neben den üblichen Hilfs- und Trägerstoffen enthalten, wie Füll- und Streckmittel, Bindemittel, Feuchthaltemittel, Sprengmittel, Lösungsverzögerer, Resorptionsbeschleuniger, Netzmittel, Adsorptionsmittel, Gleitmittel oder Gemische dieser Stoffe.

Der Wirkstoff kann gegebenenfalls mit einem oder mehreren der oben angegebenen Trägerstoffe auch in mikroverkapselter Form vorliegen.

Die therapeutisch wirksamen Verbindungen sollen in ihren pharmazeutischen Zubereitungen vorzugsweise in einer Konzentration von etwa 0,1 bis 99,5, vorzugsweise von etwa

0,5 bis 95 Gewichtsprozent der Gesamtmischung vorhanden sein.

Die oben aufgeführten pharmazeutischen Zubereitungen können neben dem erfindungsgemäßen Wirkstoff auch weitere pharmazeutische Wirkstoffe enthalten.

Im allgemeinen hat es sich als vorteilhaft erwiesen, den erfindungsgemäßen Wirkstoff in Gesamtmengen von etwa 1,2 bis etwa 2,4 g je 24 Stunden, gegebenenfalls in Form mehrerer Einzelgaben zur Erzielung der gewünschten Ergebnisse zu verabreichen. Eine Einzelgabe enthält den erfindungsgemäßen Wirkstoff vorzugsweise in Mengen von etwa 20 - 500 mg, insbesondere 50 - 100 mg.

Ende der Beschreibung

Patentansprüche

1. N,N'-Bis (5-oxo-1-methyl-pyrrolidin-2-yl-essigsäure) -hydrazid der Formel

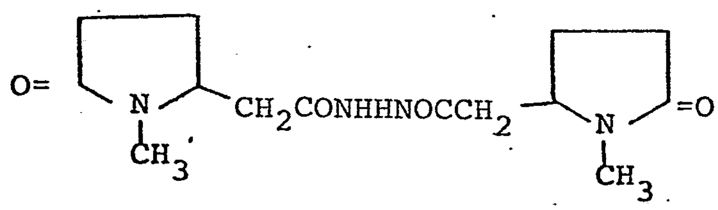

(I)

2. Verfahren zur Herstellung der Verbindung nach Anspruch 1, dadurch gekennzeichnet, daß man 1-Methyl-5-oxo-pyrrolidin-2-essigsäureester der allgemeinen Formel

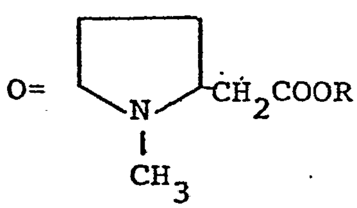

(II)

in der R eine $C_1$-$C_4$-Alkylgruppe bedeutet, mit Hydrazinhydrat zunächst zum 1-Methyl-5-oxo-pyrrolidin-2-essigsäurehydrazid und dieses Zwischenprodukt in der Wärme mit einem 1-Methyl-5-oxo-pyrrolidin-2-essigsäureester der allgemeinen Formel (II), worin R die obige Bedeutung hat, zur Verbindung der Formel (I) umsetzt.

3. Arzneimittel enthaltend die Verbindung der Formel (I) als Wirkstoff, gegebenenfalls zusammen mit üblichen Hilfs- und Trägerstoffen.

4. Verwendung von N,N'-Bis(5-oxo-1-methyl-pyrrolidin-2-yl-essigsäure)-hydrazid der Formel (I) bei der Bekämpfung cerebraler Funktionsstörungen.

Europäisches Patentamt

**EUROPÄISCHER RECHERCHENBERICHT**

0046294

Nummer der Anmeldung

EP 81 10 6387

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 2 496 163 (H.W.JACOBSON)<br>* Spalte 7, Zeilen 20-26; Patentansprüche *<br>-- | 1-4 |
| D,X | DE - B - 2 440 633 (F.W.LANGE GMBH)<br>* insgesamt *<br>-- | 1-4 |
| D,X | DE - A - 2 757 680 (TROPONWERKE)<br>* insgesamt *<br>-- | 1-4 |
| D,X | DE - A - 2 745 907 (F.W.LANGE GMBH)<br>* insgesamt *<br>-------- | 1-4 |

KLASSIFIKATION DER ANMELDUNG (Int. Cl.3)

C 07 D 207/26

RECHERCHIERTE SACHGEBIETE (Int. Cl.3)

C 07 D 207/26

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 11-09-1981 | MAISONNEUVE |